# EUROPEAN PATENT APPLICATION

(11) **EP 0 846 775 A2**
(43) Date of publication of application: **10.06.1998**
(21) Application number: 97309769.4
(22) Date of filing: 02.12.1997
(51) Int. Cl.: C12Q 1/68

(54) **Improvements and relating to indentification**

(30) Priority: 03.12.1996 GB 9625124
(71) Applicant: The Secretary of State of the Home Department, Birmingham B5 6QQ (GB)
(72) Inventor: Watson, Stephanie, The Forensic Science Service, Birmingham, B5 6QQ (GB); Kimpton, Colin, c/o The Forensic Science Service, Birmingham, B5 6QQ (GB)
(74) Representative: Pawlyn, Anthony Neil

(57) **Abstract**

DNA profiling provides an extremely useful tool in a wide variety of applications including anthropological studies, paternity investigations and in consideration of forensic evidence.

The present invention provides improved primers, primer pairs and loci selections for giving increased discriminating power in DNA profiling techniques making use of short tandem repeats (STR) or micro satellite loci. A primer pair mixture including sequences:- 
pair a)    TGC AGA TCT TGG GAC TTC TC
            AAA AAG AGT GTG GGC AAC TG
pair b)    CCA GTG GAT TTG GAA ACA GA
            ACC TAG CAT GGT ACC TGC AG
pair c)    ACT GCA GTC CAA TCT GGG T
            ATG AAA TCA ACA GAG GCT TG
pair d)    GCA ACA TTA GGT ACA TAC AAG
            AAA ATA ATC AGG GTG CTG TGT
pair e)    TAC ATC CAA ATT AGG TAG GTA GAC AA
            TGT TGA GAC ACA GCA GTA GCA T
pair f)    CTG TCT CAG ACC ATA CCT AAA TCT CT
            TCT TAG TGG GGA AGG GAT CCT AGT TAT CAC
pair g)    GTG GGC TGA AAA GCT CCC GAT TAT
            GTG ATT CCC ATT GGC CTG TTC CTC
 is provided.

SEQUENCE LISTING
(1) GENERAL INFORMATION:

(i) APPLICANT:
(A) NAME: The Secretary Of State Of The Home
Department
(B) STREET: The Forensic Science Service, Priory
House,
Gooch Street N
(C) CITY: Birmingham
(D) STATE: W Midlands
(E) COUNTRY: United Kingdom
(F) POSTAL CODE (ZIP): B5 6QQ

(ii) TITLE OF INVENTION: Improvements in and relating to
identification

(iii) NUMBER OF SEQUENCES: 28

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30
(EPO)

(vi) PRIOR APPLICATION DATA:
(A) APPLICATION NUMBER: GB 9625124.4
(B) FILING DATE: 03-DEC-1996

(2) INFORMATION FOR SEQ ID NO: 1:

(i) SEQUENCE CHARACTERISTICS:
(A) LENGTH: 20 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(I) ORGANELLE: Mitochondrion

(xi) SEQUENCE DESCRIPTION: SEQ ID NO: 1:

TGCAGATCTT GGGACTTCTC
20

(2) INFORMATION FOR SEQ ID NO: 2:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(I) ORGANELLE: Mitochondrion

(xi) SEQUENCE DESCRIPTION: SEQ ID NO: 2:

AAAAAGAGTG TGGGCAACTG
20

(2) INFORMATION FOR SEQ ID NO: 3:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: Homo sapiens
(I) ORGANELLE: Mitochondrion

(xi) SEQUENCE DESCRIPTION: SEQ ID NO: 3:

CCAGTGGATT TGGAAACAGA
20

(2) INFORMATION FOR SEQ ID NO: 4:

(i) SEQUENCE CHARACTERISTICS:
(A) LENGTH: 20 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: homo sapiens
(I) ORGANELLE: Mitochondrion

(xi) SEQUENCE DESCRIPTION: SEQ ID NO: 4:

ACCTAGCATG GTACCTGCAG
20

(2) INFORMATION FOR SEQ ID NO: 5:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: homo sapiens
(I) ORGANELLE: Mitochondrion

(xi) SEQUENCE DESCRIPTION: SEQ ID NO: 5:

ACTGCAGTCC AATCTGGGT
19

(2) INFORMATION FOR SEQ ID NO: 6:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: homo sapiens
(I) ORGANELLE: Mitochondrion

(xi) SEQUENCE DESCRIPTION: SEQ ID NO: 6:

ATGAAATCAA CAGAGGCTTG
20

(2) INFORMATION FOR SEQ ID NO: 7:

(i) SEQUENCE CHARACTERISTICS:
(A) LENGTH: 21 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: homo sapiens
(I) ORGANELLE: Mitochondrion

(xi) SEQUENCE DESCRIPTION: SEQ ID NO: 7:

GCAACATTAG GTACATACAA G
21

(2) INFORMATION FOR SEQ ID NO: 8:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: homo sapiens
(I) ORGANELLE: Mitochondrion

(xi) SEQUENCE DESCRIPTION: SEQ ID NO: 8:

AAAATAATCA GGGTGCTGTG T
21

(2) INFORMATION FOR SEQ ID NO: 9:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: homo sapiens
(I) ORGANELLE: Mitochondrion

(xi) SEQUENCE DESCRIPTION: SEQ ID NO: 9:

TACATCCAAA TTAGGTAGGT AGACAA
26

(2) INFORMATION FOR SEQ ID NO: 10:

(i) SEQUENCE CHARACTERISTICS:
(A) LENGTH: 22 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: homo sapiens
(I) ORGANELLE: Mitochondrion

(xi) SEQUENCE DESCRIPTION: SEQ ID NO: 10:

TGTTGAGACA CAGCAGTAGC AT
22

(2) INFORMATION FOR SEQ ID NO: 11:

(i) SEQUENCE CHARACTERISTICS:
(A) LENGTH: 26 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: homo sapiens
(I) ORGANELLE: Mitochondrion

(xi) SEQUENCE DESCRIPTION: SEQ ID NO: 11:

CTGTCTCAGA CCATACCTAA ATCTCT
26

(2) INFORMATION FOR SEQ ID NO: 12:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: homo sapiens
(I) ORGANELLE: Mitochondrion

(xi) SEQUENCE DESCRIPTION: SEQ ID NO: 12:

TCTTAGTGGG GAAGGGATCC TAGTTATCAC
30

(2) INFORMATION FOR SEQ ID NO: 13:

(i) SEQUENCE CHARACTERISTICS:
(A) LENGTH: 24 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: homo sapiens
(I) ORGANELLE: Mitochondrion

(xi) SEQUENCE DESCRIPTION: SEQ ID NO: 13:

GTGGGCTGAA AAGCTCCCGA TTAT
24

(2) INFORMATION FOR SEQ ID NO: 14:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: homo sapiens
(I) ORGANELLE: Mitochondrion

(xi) SEQUENCE DESCRIPTION: SEQ ID NO: 14:

GTGATTCCCA TTGGCCTGTT CCTC
24

(2) INFORMATION FOR SEQ ID NO: 15:

(i) SEQUENCE CHARACTERISTICS:
(A) LENGTH: 30 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 15:

CCCTAGTGGA TGATAAGAAT AATCAGTATG
30

(2) INFORMATION FOR SEQ ID NO: 16:

(i) SEQUENCE CHARACTERISTICS:
(A) LENGTH: 30 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 16:

GGACAGATGA TAAATACATA GGATGGATGG
30

(2) INFORMATION FOR SEQ ID NO: 17:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 17:

GTGGGCTGAA AAGCTCCCGA TTAT
24

(2) INFORMATION FOR SEQ ID NO: 18:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 18:

GTGATTCCCA TTGGCCTGTT CCTC
24

(2) INFORMATION FOR SEQ ID NO: 19:

(i) SEQUENCE CHARACTERISTICS:
(A) LENGTH: 22 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 19:

ATATGTGAGT CAATTCCCCA AG
22

(2) INFORMATION FOR SEQ ID NO: 20:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 20:

TGTATTAGTC AATGTTCTCC AG
22

(2) INFORMATION FOR SEQ ID NO: 21:

(i) SEQUENCE CHARACTERISTICS:
(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 21:

CAAACCCGAC TACCAGCAAC
20

(2) INFORMATION FOR SEQ ID NO: 22:

(i) SEQUENCE CHARACTERISTICS:
(A) LENGTH: 20 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 22:

GAGCCATGTT CATGCCACTG
20

(2) INFORMATION FOR SEQ ID NO: 23:

(i) SEQUENCE CHARACTERISTICS:
(A) LENGTH: 21 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 23:

GCCCCATAGG TTTTGAACTC A
21

(2) INFORMATION FOR SEQ ID NO: 24:

(i) SEQUENCE CHARACTERISTICS:
(A) LENGTH: 22 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 24:

TGATTTGTCT GTAATTGCCA GC
22

(2) INFORMATION FOR SEQ ID NO: 25:

(i) SEQUENCE CHARACTERISTICS:
(A) LENGTH: 25 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 25:

TTTTTGTATT TCATGTGTAC ATTCG
25

(2) INFORMATION FOR SEQ ID NO: 26:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 26:

CGTAGCTATA ATTAGTTCAT TTTC
24

(2) INFORMATION FOR SEQ ID NO: 27:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 27:

CCCTGGGCTC TGTAAAGAAT AGTG
24

(2) INFORMATION FOR SEQ ID NO: 28:

(i) SEQUENCE CHARACTERISTICS:
(A) LENGTH: 24 base pairs
(B) TYPE: nucleic acid
(C) STRANDEDNESS: single

(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:
(A) ORGANISM: HOMO SAPIENS
(I) ORGANELLE: Mitochondrion

(xi) SEQUENCE DESCRIPTION: SEQ ID NO: 28:

ATCAGAGCTT AAACTGGGAA GCTG
24

## Description

This invention is concerned with improvements in and relating to identification, particularly, but not exclusively to identification and/or analysis of DNA samples.

DNA profiling provides an extremely useful tool in a wide variety of applications including anthropological studies and paternity investigations. It is particularly useful in considering forensic evidence, for instance of the type found at the scene of a crime. Profiles can be used to demonstrate a link, or a lack of a link, between samples.

One of the most versatile DNA profiling techniques makes use of short tandem repeats (STR) or micro satellite loci. These are a class of polymorphic markers which consist of simple tandemly repeated sequences of between 1 and 6 base pairs in length throughout non-coding parts of the human genome. On average STRs occur every 6 to 10 Kilo bases along the DNA. The length displays a high degree of variation, hyper-variability, due to differences in the number of repeat units displayed by differing individuals. This degree of variability provides the distinguishing power to the profile technique.

STRs represent markers which can be used in identifying features of interest in a DNA sequence or in comparing the equivalence of sequences from different sources.

The present invention seeks to provide improved primers for use with such a profiling system, an improved set of primers for use with such a system and improved discrimination between samples, amongst other aims.

According to a first aspect of the invention we provide a mixture comprising at least three primer pairs consisting of or comprising the sequences :- 
pair a)    TGC AGA TCT TGG GAC TTC TC
           AAA AAG AGT GTG GGC AAC TG
pair b)    CCA GTG GAT TTG GAA ACA GA
 
ACC TAG CAT GGT ACC TGC AG
pair c) ACT GCA GTC CAA TCT GGG T
ATG AAA TCA ACA GAG GCT TG
pair d) GCA ACA TTA GGT ACA TAC AAG
AAA ATA ATC AGG GTG CTG TGT
pair e) TAC ATC CAA ATT AGG TAG GTA GAC AA
TGT TGA GAC ACA GCA GTA GCA T
pair f) CTG TCT CAG ACC ATA CCT AAA TCT CT
TCT TAG TGG GGA AGG GAT CCT AGT TAT CAC
pair g) GTG GGC TGA AAA GCT CCC GAT TAT
GTG ATT CCC ATT GGC CTG TTC CTC
 or primer pairs consisting of or comprising sequences at least 75% homologous thereto.

Preferably at least one primer in each primer pair is detectably labelled. Labelling before use is preferred. Dye labels are preferred, but other label forms, such as radioactive isotopes, maybe employed. A fluorescent dye, such as phosphodiester dyes, may be used. Such dyes include those designated 6-FAM, TET, TAMRA or HEX amidites.

Preferably the primers relating to STR's of overlapping size ranges are differently labelled.

Preferably a primer from pair a has a different label to primers from pairs b, d or g. Preferably a primer from pair b has a different label to primers from pairs a and g. Preferably a primer from pair d has a different label to primers from pairs a, e or f. Preferably a primer from pair e has a different label to primers from pairs d or f. Preferably a primer from pair f has a different label to primers from pairs d or e. Preferably a primer from pair g has a different label to primers from pairs a or b.

Preferably pairs a and e are provided with a first label, paris b and f with a second label and pairs d and g with a third label. Pair c may have the first, second or third label, the first is preferred.

The mixture may also contain the deoxynucleoside triphosphates, dNTP's; dATP, dCTP, dGTP and dTTP.

The mixture may be provided in a buffer, for instance Tris-HCl or Tris-HCl together with KCl and MgCl₂.

Preferably at least 4, 5, 6 and even more preferably 7 of the primer pairs are provided in the mixture. The level of discrimination increases with the number or primer pairs employed.

Preferably the sequence is provided in an overall sequence of less than 75 nucleotides. More preferably the sequence of between 15 and 35 nucleotides.

Preferably the primers and primer pairs are at least 90% homologous with the listed sequences. More preferably the primers included the sequences stated above.

According to a second aspect of the invention we provide a mixture of primers pairs, wherein the primers pairs hybridised to four or more of the following loci pairs:-

| | Locus | Chromosome |
|---|---|---|
| pair a) | D1S518 I | 1 |
| | D1S518 II | 1 |
| pair b) | GGAA3A09 I | 2 |
| | GGAA3AO9 II | 2 |
| pair c) | D3S1358 I | 3 |
| | D3S1358 II | 3 |
| pair d) | D10S516 I | 10 |
| | D10S516 II | 10 |
| pair e) | D14S306 I | 14 |
| | D14S306 II | 14 |
| pair f) | GATA4F03 I | 22 |
| | GATA4F03 II | 22 |
| pair g) | HUMTHO1 I | 11 |
| | HUMTHO1 II | 11 |

The loci are named according to the gene bank designations.

Preferably primers hybridising to all seven loci are provided in the mixture.

According to a third aspect of the invention we provide one or more primers consisting of, or comprising, one or more of the following sequences, or being 75% homologous thereto:-

i) TAC ATC CAA ATT AGG TAG GTA GAC AA
ii) TGT TGA GAC ACA GCA GTA GCA T
iii) CTG TCT CAG ACC ATA CCT AAA TCT CT
iv) TCT TAG TGG GGA AGG GAT CCT AGT TAT CAC

Preferably the invention provides one or more pairs of primers consisting of, or comprising the listed sequences, or 75% homologous thereto, in pairs i),ii) and / or iii) and iv).

Preferably a mixture of primers is provided, the mixture involving two or more primers or one or more primer pairs, as listed.

According to a fourth aspect of the invention we provide a primer hybridising to one or more of the following loci:- D10S516 I, D10S516 II, D145306 I, D145306 II, GATA4F03 I or GATA4F03 II.

According to a fifth aspect of the invention we provide a kit comprising a mixture of primers pairs according to the first and/or second aspect of the invention and/or including primers according to the third and/or fourth aspect of the invention, together with a buffer and deoxynucleoside triphosphates.

The kit may further provide DNA polymerase, for instance Taq polymerase, and/or a positive control sample to the primers provided and/or a reference mixture and/or restriction enzymes.

The deoxynucleoside triphosphates preferably include dATP, dCTP, dGTP and dTTP.

According to a sixth aspect of the invention we provide a method of investigation a DNA sample comprising :-
obtaining a sample containing DNA to be analysed;
contacting the DNA with a DNA polymerase and with a primer mixture, the primer mixture comprising four or more primer pairs consisting of, or comprising the following sequences, or primer pairs 75% homologous therewith, 
pair a)    TGC AGA TCT TGG GAC TTC TC
           AAA AAG AGT GTG GGC AAC TG
pair b)    CCA GTG GAT TTG GAA ACA GA
           ACC TAG CAT GGT ACC TGC AG
pair c)    ACT GCA GTC CAA TCT GGG T
           ATG AAA TCA ACA GAG GCT TG
pair d)    GCA ACA TTA GGT ACA TAC AAG
           AAA ATA ATC AGG GTG CTG TGT
pair e)    TAC ATC CAA ATT AGG TAG GTA GAC AA
           TGT TGA GAC ACA GCA GTA GCA T
pair f)    CTG TCT CAG ACC ATA CCT AAA TCT CT
           TCT TAG TGG GGA AGG GAT CCT AGT TAT CAC
pair g)    GTG GGC TGA AAA GCT CCC GAT TAT
           GTG ATT CCC ATT GGC CTG TTC CTC
or with one or more primers comprising or consisting of sequences :- 
i)    TAC ATC CAA ATT AGG TAG GTA GAC AA
ii)   TGT TGA GAC ACA GCA GTA GCA T
iii)  CTG TCT CAG ACC ATA CCT AAA TCT CT
iv)   TCT TAG TGG GGA AGG GAT CCT AGT TAT CAC
 and amplifying the mixture and obtaining an indication of the constituent parts of the sample analysed.

Preferably the DNA sample is one or more of a sample taken from a scene of crime, a sample obtained from a suspect, a sample obtained from an organism under investigation or a reference sample. The sample containing the DNA may be a blood sample, a bodily fluid sample or of other type including skin and hair samples or those used in paternity investigations.

Preferably the DNA is provided in the sample between 0.5 and 10 ng/ul and more preferably between 1 and 3 ng/ul.

Preferably the DNA sample is suspended in between 1 and 50 ul of sterile distilled water. 30 ul is particularly preferred.

Preferably between 0.13 and 0.37 ul of DNA polymerase, most preferably Taq polymerase is provided. 0.25 ul provides a particularly preferred volume.

Preferably between 5 and 30 ul of the primer mixture is provided. 19.75 ul is a particularly preferred value. The primer may be provided in a suitable buffer.

Preferably the application of the DNA is carried out by PCR. Preferably three cycle files are used. Preferably these include one or more of, and most preferably consist of :-
a three temperature cycle file; 94°C + / - 4°C for 60 seconds, + / - 60 seconds; 58°C + / - 4°C for 60 seconds, + / - 60 seconds; and 72°C + / - 4°C for 60 seconds, + / - 60 seconds; repeated for 28 cycles, + / - 5 cycles;
a one temperature cycle file, 72°C for 30 minutes, + / - 30 minutes; and
a 4°C soak file.

Preferably the files are linked in the order listed above.

Preferably the amplified products are separated, most preferably according to their size. The products may be separated by gel electrophoresis. Preferably polyacrylamide gel electrophoresis is used.

The fluorescent dye labels may be detected by laser application to the gel results. Most preferably two or more of the different primers are detected as different colours. Most preferably their intensities are also determined.

Preferably the method includes comparison of a DNA sample obtained from a suspect with a DNA sample obtained from the scene of a crime. Preferably these results are further compared with DNA profile results from a database, such as the National DNA Database. Automated comparison, for instance by computers, is particularly facilitated by STR analysis as STR's can readily be designated as a string of numerical digits, based on the number of repeat units.

Alternatively, the method may comprise the comparison of a DNA sample from one organism with another, for instance to determine the hereditary relationship between them. Thus the technique may be used in paternity or for other relationship determinations.

The method may further include the investigation of an equivalent DNA sample with one or more other primers or primer mixtures.

It is particularly preferred that the method include analysis of an equivalent sample using one or more primer pairs comprising or consisting of sequences:- 
pair a)    CCC TAG TGG ATG ATA AGA ATA ATC AGT ATG
            GGA CAG ATG ATA AAT ACA TAG GAT GGA TGG
pair b)    GTG GGC TGA AAA GCT CCC GAT TAT
            GTG ATT CCC ATT GGC CTG TTC CTC
pair c)    ATA TGT GAG TCA ATT CCC CAA G
            TGT ATT AGT CAA TGT TCT CCA G
pair d)    CAA ACC CGA CTA CCA GCA AC
            GAG CCA TGT TCA TGC CAC TG
pair e)    GCC CCA TAG GTT TTG AAC TCA
            TGA TTT GTC TGT AAT TGC CAG C
pair f)    TTT TTG TAT TTC ATG TGT ACA TTC G
            CGT AGC TAT AAT TAG TTC ATT TTC
pair g)    CCC TGG GCT CTG TAA AGA ATA GTG
            ATC AGA GCT TAA ACT GGG AAG CTG
 or one or more primer hybridising to one or more of the following loci:-
a) HUMVWFA31
b) HUMTH01
c) D21511
d) D18551
e) HUMFIBRA
f) D851179
g) HUMAMGXA and / or HUMAMGY

It is particularly preferred that the determination of the results be conducted on a Perkin Elmer (Applied Biosystems) device and most particularly a A30 373 Prism sequencer device or a 377 device.

The results of the method can be further processed or analysed using statistical techniques, for instance comparison with a database providing frequency of occurrence information for the constituents detected in the population as a whole, or a representative sample.

Various embodiments of the invention will now be described, by way of example only, and with reference to the accompanying drawings, in which:
Figure 1 shows the primer sequences of a mixture according to one embodiment of the invention;
Figure 2 shows the chromosome, locus and STR size for the sequences of Figure 1;
Figure 3 shows suitable dye label structures; and
Figure 4 shows an additional set of primer sequences of a mixture for use in the present invention.

The present invention can be illustrated by means of an STR profiling system provided in the following way.

The STR primers are provided in a 1 ml aliquot, together with PARR buffer (100mM Tris-HCl pH 8.3, 500mM KCl, 15mM MgCl₂, 1% Triton) and the necessary dNTP's, dATP, dCTP, dGTP and dTTP. Perkin Elmer Buffer 1 (100mM Tris-HCl pH 8.3, 500mM KCl, 15mM MgCl₂, 0.01% gelatine) may be used as an alternative.

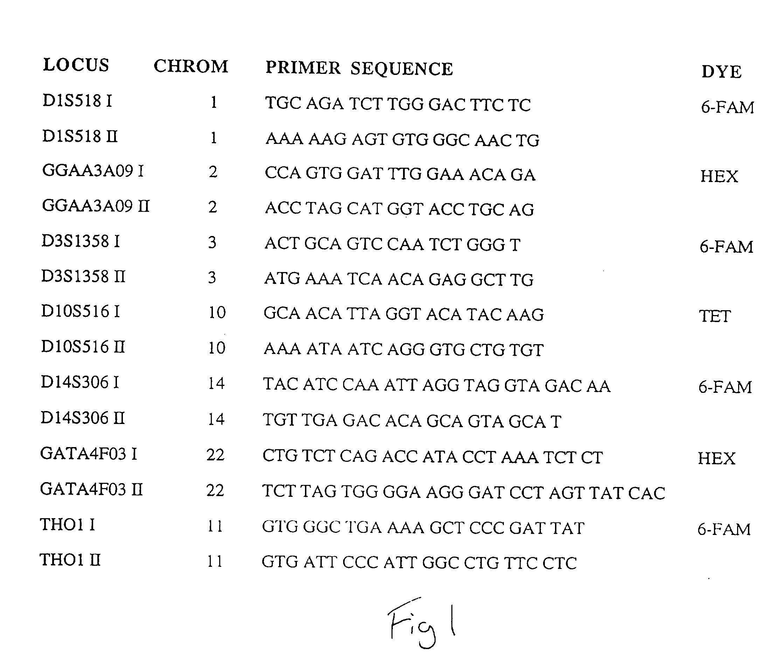

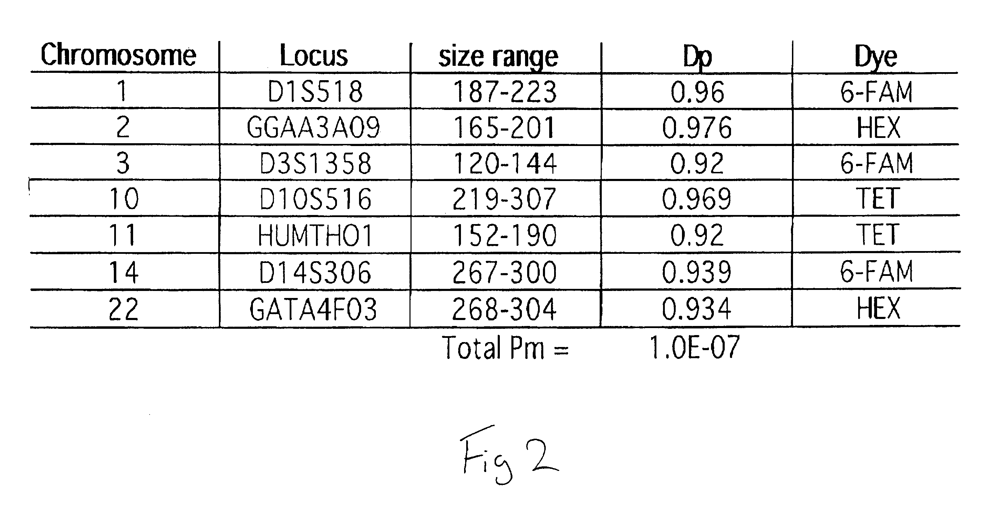

The primer sequences employed were illustrated in Figure 1.

Prior to use the aliquot is kept frozen at below 20°C and once unfrozen is stored at 4°C or less. The aliquot is used within a month of freezing and repeated freeze thaw is avoided.

The DNA sample to be analysed can be obtained in a variety of ways. To ensure accuracy in the results the samples should be carefully selected, stored and prepared.

### Control Samples

Blood samples should be obtained from each person in question and taken up into Monovette syringes, or equivalent, treated with the anticoagulant EDTA and stored frozen. If it is not possible to obtain a blood sample then the following may be suitable as controls for DNA Profiling tests.

Plucked head hairs - a minimum of twenty hairs with visible root material from each individual.

Buccal swabs - (buccal swabs are rubbed around the inside of the mouth removing cells from the lining of the cheeks) - at least two swabs from each individual.

### Handling Storage of Crime Samples

Liquid blood samples at scenes should be collected into clean sterile containers and frozen.

Liquid semen samples, although rarely encountered at a scene, should be treated in the same way as liquid blood. A condom containing semen should be frozen together with its contents. It might be possible to obtain a DNA Profile from the vaginal material on the outside of the condom as well as from the semen.

Swabs - vaginal, anal and oral. To maximise the recovery of semen, duplicate swabs should be taken from the appropriate areas and stored frozen.

Penile swabs may on occasion provide sufficient vaginal material for DNA Profiling. As there is likely to be only limited material present only one swab should be taken, moistened with sterile filtered water before use, then frozen.

Swabs from other areas - semen on the face, body or other surfaces should be removed using a swab previously moistened with sterile filtered water. An adjacent area free from seme should be identified as a "control" and a similar swab taken. Store frozen.

Wet or stained clothing - air dry and pack in brown paper bags, handle as little as possible.

Hair/cigarette butts - pack in bag or clean sterile container and keep cool or freeze.

Bone/muscle/flesh/skin - collected into clean sterile containers and frozen.

For use in the amplification procedure the DNA should be provided as between a 1 and 3 ng sample which is suspended in 30 ul of sterile distilled water. The sample is then mixed with 19.75 ul of the primer mixture and 0.25 ul of Taq polymerase to give a 50 ul volume reaction mixture.

The amplification is carried out using a PCR unit with a drop of mineral oil being provided over the reaction mixture during cycling to prevent evaporation. A P.E. Thermal cycler 9600 unit is used for amplification and three cycle files are used. The first consists of a three temperature cycle file, 94°C for 60 seconds, 58°C for 60 seconds and 72°C for 60 seconds repeated for 28 cycles. The second cycle is a single temperature cycle at 72°C for 30 minutes. The third comprises a soak file maintained at 4°C. The three files are linked together in order 1, 2, 3.

Following amplification the products are stored at 4°C until the electrophoresis is carried out.

The substitution of Amplitac gold TM (obtained from Perkin Elmer) for the Taq polymerase is possible with the addition of a precycle of 95°C for 12 minutes before the first temperature cycle set out above.

The amplified samples are segregated on the basis of their size using polyacrylamide gel electrophoresis.

A different coloured dye is attached to one of the primers in pairs a, c, e; b, f; d, g as listed above, so providing a readily monitorable different colour response.

A series of validation reagents and reference materials may be employed in confirming or validating the results obtained. A DNA positive test may be provided using a chelex extraction and quantitated using the Dot blot human alphasatellite probe or a picogreen assay. As an additional safeguard an allelic ladder may be produced providing a reference standard to designate alleles with standard nomenclature. A quality control test to ensure that the new cocktail matches the reference allelic ladder for the allele content and to maintain the concentration of the ladder required for use is thus provided.

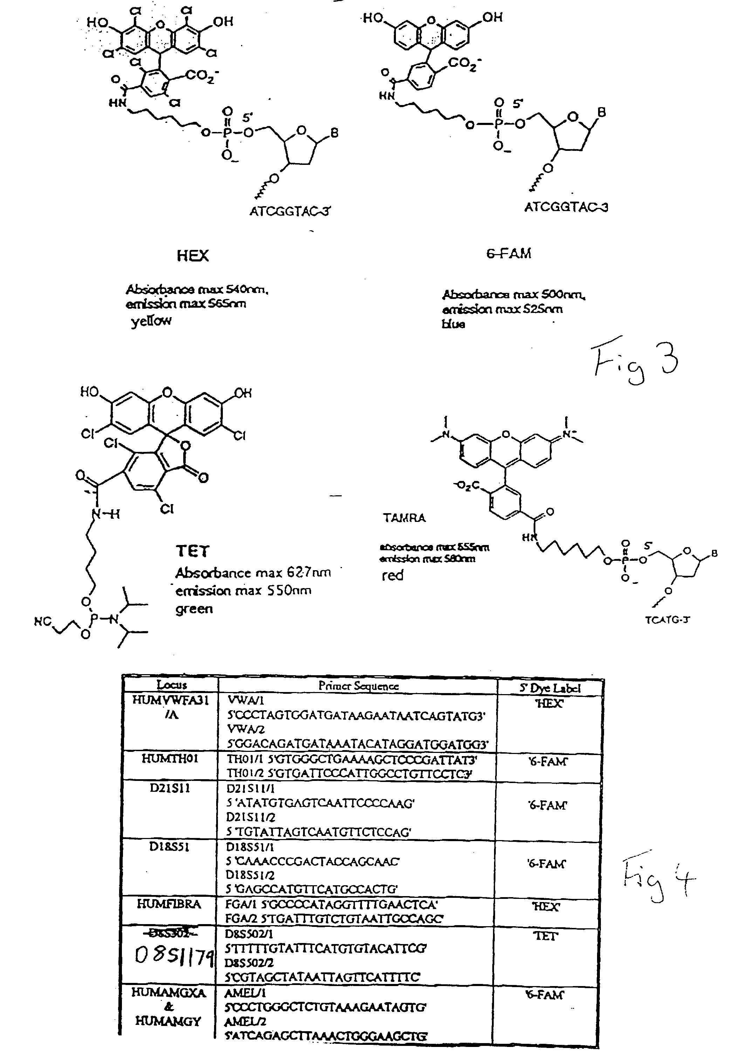

To provide additional discrimination a sample of the DNA obtained prepared in an equivalent manner may also be subjected to similar amplification and analysis procedure employing an alternative set of primers. Figure 2 illustrates an alternative set of seven primer pairs suitable for this purpose.

Each of the two sets of primer pairs proposed provides discriminating power of approximately 1 in 50 million. Thus in combination discriminating power of 1 in 2.5d x 10¹⁵ is provided.

The reaction system by its provision in a single tube is both rapid and cost effective.

The primers selected are addressed to loci which provide a high level of discrimination in each case, a match probability of <0.1. Additionally a high degree of heterozygosity is achieved, at least 0.7.

The provision of the system at between 100 and 349 base pairs in length through selection and design is also achieved.

Each of the primers also amplifies well individually and when present in combination with the other primers / loci.

## Claims

1. A mixture comprising at least three primer pairs consisting of or comprising the sequences :- 
pair a)    TGC AGA TCT TGG GAC TTC TC
           AAA AAG AGT GTG GGC AAC TG
pair b)    CCA GTG GAT TTG GAA ACA GA
           ACC TAG CAT GGT ACC TGC AG
pair c)    ACT GCA GTC CAA TCT GGG T
           ATG AAA TCA ACA GAG GCT TG
pair d)    GCA ACA TTA GGT ACA TAC AAG
           AAA ATA ATC AGG GTG CTG TGT
pair e)    TAC ATC CAA ATT AGG TAG GTA GAC AA
           TGT TGA GAC ACA GCA GTA GCA T
pair f)    CTG TCT CAG ACC ATA CCT AAA TCT CT
           TCT TAG TGG GGA AGG GAT CCT AGT TAT CAC
pair g)    GTG GGC TGA AAA GCT CCC GAT TAT
           GTG ATT CCC ATT GGC CTG TTC CTC
 or primer pairs consisting of or comprising sequences at least 75% homologous thereto.

2. A mixture according to claim 1 in which at least one primer in each primer pair is detectably labelled and in which the primers relating to STR's of overlapping size ranges are differently labelled.

3. A mixture according to claim 1 or claim 2 in which a primer from pair a has a different label to primers from pairs b, d or g, and / or a primer from pair b has a different label to primers from pairs a and g, and / or a primer from pair d has a different label to primers from pairs a, e or f and / or a primer from pair e has a different label to primers from pairs d or f and / or a primer from pair f has a different label to primers from pairs d or e and / or a primer from pair g has a different label to primers from pairs a or b.

4. A mixture according to any of claims 1 to 3 in which pairs a and e are provided with a first label, pairs b and f with a second label and pairs d and g with a third label.

5. A mixture according to any of claims 1 to 4 in which at least 5 and preferably 7 of the primer pairs are provided in the mixture.

6. A mixture according to any of claims 1 to 5 in which the specified sequence or sequences are provided in an overall sequence of between 15 and 35 nucleotides.

7. A mixture according to any of claims 1 to 6 in which the primers and / or primer pairs are at least 90% homologous with the listed sequences.

8. A mixture of primers pairs, wherein the primers pairs hybridised to four or more of the following loci pairs:-
| | Locus | Chromosome |
|---|---|---|
| pair a) | D1S518 I | 1 |
| | D1S518 II | 1 |
| pair b) | GGAA3A09 I | 2 |
| | GGAA3A09 II | 2 |
| pair c) | D3S1358 I | 3 |
| | D3S1358 II | 3 |
| pair d) | D10S516 I | 10 |
| | D10S516 II | 10 |
| pair e) | D14S306 I | 14 |
| | D14S306 II | 14 |
| pair f) | GATA4F03 I | 22 |
| | GATA4F03 II | 22 |
| pair g) | HUMTHO1 I | 11 |
| | HUMTHO1 II | 11 |

9. A mixture according to claim 8 in which primers hybridising to all seven loci are provided in the mixture.

10. One or more primers consisting of, or comprising, one or more of the following sequences, or being 75% homologous thereto:-

i)    TAC ATC CAA ATT AGG TAG GTA GAC AA
ii)   TGT TGA GAC ACA GCA GTA GCA T
iii)  CTG TCT CAG ACC ATA CCT AAA TCT CT
iv)   TCT TAG TGG GGA AGG GAT CCT AGT TAT CAC

11. A primer hybridising to one or more of the following loci:-
D10S516 I, D10S516 II, D145306 I, D145306 II, GATA4F03 I or GATA4F03 II.

12. A kit comprising a mixture of primers pairs according to any of claims 1 to 9 or including primers according to claim 10 and / or claim 11 together with a buffer and deoxynucleoside triphosphates.

13. A kit according to claim 12, the kit further providing DNA polymerase, for instance Taq polymerase, and/or a positive control sample to the primers provided and/or a reference mixture and/or restriction enzymes.

14. A method of investigation a DNA sample comprising:-
obtaining a sample containing DNA to be analysed; contacting the DNA with a DNA polymerase and with a primer mixture, the primer mixture comprising four or more primer pairs consisting of, or comprising the following sequences, or primer pairs 75% homologous therewith, 
pair a)    TGC AGA TCT TGG GAC TTC TC
           AAA AAG AGT GTG GGC AAC TG
pair b)    CCA GTG GAT TTG GAA ACA GA
           ACC TAG CAT GGT ACC TGC AG
pair c)    ACT GCA GTC CAA TCT GGG T
           ATG AAA TCA ACA GAG GCT TG
pair d)    GCA ACA TTA GGT ACA TAC AAG
           AAA ATA ATC AGG GTG CTG TGT
pair e)    TAC ATC CAA ATT AGG TAG GTA GAC AA
           TGT TGA GAC ACA GCA GTA GCA T
 
pair f)    CTG TCT CAG ACC ATA CCT AAA TCT CT
            TCT TAG TGG GGA AGG GAT CCT AGT TAT CAC
pair g)    GTG GGC TGA AAA GCT CCC GAT TAT
            GTG ATT CCC ATT GGC CTG TTC CTC
 or with one or more primers comprising or consisting of sequences :- 
i)    TAC ATC CAA ATT AGG TAG GTA GAC AA
ii)   TGT TGA GAC ACA GCA GTA GCA T
iii)  CTG TCT CAG ACC ATA CCT AAA TCT CT
iv)   TCT TAG TGG GGA AGG GAT CCT AGT TAT CAC
 and amplifying the mixture and obtaining an indication of the constituent parts of the sample analysed.

15. A method according to claim 14 in which the DNA sample is one or more of a sample taken from a scene of crime, a sample obtained from a suspect, a sample obtained from an organism under investigation or a reference sample.

16. A method according to claims 14 or claim 15 in which the amplification of the DNA is carried out by PCR using three cycle files.

17. A method according to claim 16 in which the cycle file(s) include one or more of:-
a three temperature cycle file; 94°C + / - 4°C for 60 seconds, + / - 60 seconds; 58°C + / - 4°C for 60 seconds, + / - 60 seconds; and 72°C + / - 4°C for 60 seconds, + / - 60 seconds; repeated for 28 cycles, + / - 5 cycles;
a one temperature cycle file, 72°C for 30 minutes, + / - 30 minutes; and
a 4°C soak file.

18. A method according to any of claims 14 to 17 in which the method includes comparison of a DNA sample obtained from a suspect with a DNA sample obtained from the scene of a crime.

19. A method according to any of claims 14 to 17 in which the method may comprise the comparison of a DNA sample from one organism with another to determine the hereditary relationship between them.

20. A method according to any of claims 14 to 18 in which the method further includes the investigation of an equivalent DNA sample with one or more other primers or primer mixtures.

21. A method according to any of claims 14 to 20 in which the method includes analysis of an equivalent sample using one or more primer pairs comprising or consisting of sequences:- 
pair a)    CCC TAG TGG ATG ATA AGA ATA ATC AGT ATG
           GGA CAG ATG ATA AAT ACA TAG GAT GGA TGG
pair b)    GTG GGC TGA AAA GCT CCC GAT TAT
           GTG ATT CCC ATT GGC CTG TTC CTC
pair c)    ATA TGT GAG TCA ATT CCC CAA G
           TGT ATT AGT CAA TGT TCT CCA G
pair d)    CAA ACC CGA CTA CCA GCA AC
           GAG CCA TGT TCA TGC CAC TG
pair e)    GCC CCA TAG GTT TTG AAC TCA
           TGA TTT GTC TGT AAT TGC CAG C
pair f)    TTT TTG TAT TTC ATG TGT ACA TTC G
           CGT AGC TAT AAT TAG TTC ATT TTC
pair g)    CCC TGG GCT CTG TAA AGA ATA GTG
           ATC AGA GCT TAA ACT GGG AAG CTG
 or one or more primer hybridising to one or more of the following loci:-
a) HUMVWFA31
b) HUMTH01
c) D21511
d) D18551
e) HUMFIBRA
f) D851179
g) HUMAMGXA and / or HUMAMGY

22. A method according to any of claims 14 to 21 in which the results of the method are further processed or analysed using statistical techniques, for instance comparison with a database providing frequency of occurrence information for the constituents detected in the population as a whole, or a representative sample.
